Europäisches Patentamt

European Patent Office

Office européen des brevets

(11) Veröffentlichungsnummer: **0 513 471 A2**

# EUROPÄISCHE PATENTANMELDUNG

(21) Anmeldenummer: **92100368.7**

(22) Anmeldetag: **11.01.92**

(51) Int. Cl.5: **A61B 17/28**, A61B 17/32

(30) Priorität: **19.04.91 DE 4112818**
**24.07.91 DE 9109113 U**

(43) Veröffentlichungstag der Anmeldung:
**19.11.92 Patentblatt 92/47**

(84) Benannte Vertragsstaaten:
**AT BE CH DE DK ES FR GB IT LI NL PT SE**

(71) Anmelder: **Kothe, Lutz**
**Bodmaner Strasse 17**
**W-7760 Radolfzell 14(DE)**

(72) Erfinder: **Kothe, Lutz**
**Bodmaner Strasse 17**
**W-7760 Radolfzell 14(DE)**

(74) Vertreter: **Weiss, Peter, Dr. rer. nat.**
**Dr. Peter Weiss & Partner Postfach 12 50**
**Zeppelinstrasse 4**
**W-7707 Engen/Hegau(DE)**

(54) **Chirurgisches Instrument.**

(57) Bei einem chirurgischen Instrument, insbesondere für die Endoskopie mit einem schließbaren Maul aus Maulteilen, wie Zangen- oder Scherenschenkel, welche an einem Zugelement (2) festgelegt sind, soll zumindest ein Maulteil (7, 8) eine Drehachse (l6, 29) bilden und um diese Drehachse drehbar angeordnet sein. Dabei durchzieht das Zugelement (2) ein Schaftrohr (l). Zumindest einem Maulteil (7, 8) soll von außen her eine Ausnehmung (l4, l4a) eingeformt sein, deren Mittelpunkt (M) um die Drehachse (l6, 29) am Zugelement (2) dreht. Die Ausnehmung (l4, l4a) umfängt in Gebrauchslage ein in der Wand des Schaftrohres (l) vorgesehenes Ringstück (25), um welches das Maulteil (7, 8) bei seiner Schließ- oder Öffnungsbewegung dreht.

Fig.1

EP 0 513 471 A2

Die Erfindung betrifft ein chirurgisches Instrument insbesondere für die Endoskopie mit einem schließbaren Maul aus Maulteilen, wie Zangen- oder Scherenschenkel, welche an einem Zugelement festgelegt sind, wobei zumindest ein Maulteil eine Drehachse mit dem Zugelement bildet und um diese Drehachse drehbar angeordnet ist und wobei das Zugelement ein Schaftrohr zumindest teilweise durchzieht.

Derartige chirurgische Instrumente sind als Zangen, Scheren, Klemmen od. dgl. in vielfältigen Ausführungsformen bekannt und dienen den unterschiedlichsten Zwecken. In der Regel bestehen sie aus zwei Maulteilen, welche relativ zueinander bewegt werden, wobei dann ein Klemmen, Schneiden oder Scheren erfolgt.

Nur beispielhaft wird auf die DE-OS 39 21 935 verwiesen, aus welcher eine Endoskopiezange bekannt ist. Die Zangenschenkel sind dort über einen Drehbolzen miteinander verbunden. Andererseits dieses Drehbolzens ist an die Zangenschenkel ein Zugseil, eine Zustange od. dgl. angekoppelt. Mit dem Zugseil ist ein Führungselement verbunden, welches zumindest einen Führungskanal aufweist, in welchen ein Hebelansatz eingreift, welcher einem Zangenschenkel jenseits des Drehbolzens bzw. eines Drehpunktes angeformt ist. Wird an diesem Führungselement gezogen, so schließt oder öffnet sich das durch die Zangenschenkel gebildete Maul.

Nachteilig bei diesen bisher bekannten Zangen ist vor allem, daß sie nach Gebrauch nur unter erheblichen Mühen auseinander genommen, gereinigt und sterilisiert werden können. Auch der Zusammenbau erfordert wiederum einen hohen Zeitaufwand bzw. ist mit einem Berühren der an sich steril zu haltenden Teile verbunden.

Ferner läßt bei vielen chirurgischen Instrumenten diese Art die Bewegungsmechanik sehr zu wünschen übrig, vor allem, wenn es um die Kraftübertragung auf die Maulteile geht.

Weiterhin sind aus den deutschen Gebrauchsmustern 87 12 328.2 und 88 14 560.3 Endoskopiezangen bekannt, bei denen die Maulteile mit dem Zugelement eine Drehachse bilden. Die Führung dieser Maulteile erfolgt in entsprechenden Wanddurchbrechungen des Schaftrohres, was allerdings sehr ungenau ist. Auch die Kraftübertragung zum Öffnen bzw. Schließen des Maules läßt sehr zu wünschen übrig. Ferner muß zur Reinigung die obere Hülse von der Spirale abgedreht werden.

Der vorliegenden Erfindung liegt die Aufgabe zugrunde, eine möglichst perfekte Bewegungsmechanik bei optimaler Kraftübertragung auf die Maulteile zu schaffen, wobei gleichzeitig ein außerordentlich leichtes und wenig zeitaufwendiges Auseinandernehmen des chirurgischen Instrumentes möglich ist.

Zur Lösung dieser Aufgabe führt, daß zumindest einem Maulteil von außen her eine Ausnehmung eingeformt ist, deren Mittelpunkt um die Drehachse am Zugelement dreht, wobei die Ausnehmung in Gebrauchslage ein in der Wand des Schaftrohres vorgesehenes Ringstück zumindest teilweise umfängt und das Maulteil bei seiner Schließ- oder Öffnungsbewegung um dieses Ringstück dreht.

Das Zusammenspiel zwischen dem Ringstück im Schaftrohr und der Ausnehmung ist von besonderer Bedeutung. Sobald das Zugelement sich in dem Schaftrohr bewegt, wird diese axiale Bewegung durch ein Gleiten der Ausnehmung um das Ringstück in eine Drehbewegung des bzw. der Maulteile umgesetzt. Somit gleiten zumindest Teile der Ausnehmung immer an den Wänden des Ringstückes entlang.

Damit nicht nur eine einseitige Bewegung des Maulteiles möglich ist, sollte die Ausnehmung etwa halbkreisförmig ausgebildet sein. Bei einem Zug am Zugelement wird hierdurch das Maulteil geschlossen, während in entgegengesetzter Richtung das Maulteil geöffnet wird.

Damit ein gutes Zusammenspiel zwischen Ringstück und Ausnehmung gewährleistet ist, sollte der Radius der Ausnehmung nur geringfügig größer sein, als der Radius des Ringstückes. In Gebrauchslage liegen somit sowohl der Mittelpunkt der Ausnehmung als auch der Mittelpunkt des Ringstückes nahe beieinander, so daß möglichst viel Flächenberührung zwischen Ringstück und Ausnehmung vorhanden ist. Hierdurch wird die Führung des Maulteiles verbessert.

In einem Ausführungsbeispiel der Erfindung kann ein Maulteil feststehend ausgebildet sein, während sich das andere Maulteil relativ zu diesem feststehenden Maulteil bewegt. Beide sind über den oben genannten Gelenkbolzen miteinander verbunden. Hier genügt es selbstverständlich, wenn in dem bewegbaren Maulteil die Ausnehmung vorgesehen ist und wenn das Schaftrohr nur einen Durchbruch oder eine Nut und nur ein ensprechendes Ringstück zum Eingleiten in die Ausnehmung besitzt. Allerdings sollte dann das feststehende Maulteil eine gesonderte Ausnehmung besitzen, in welche der Führungsschenkel des bewegbaren Maulteiles verschwinden kann.

In den bevorzugten Ausführungsbeispielen sind allerdings beide Maulteile bewegbar und somit indentisch ausgebildet, wobei dann auch zwei entsprechende Durchbrüche oder Nuten und zwei entsprechende Ringstücke in der Schaftwand angeordnet sind.

Zur Befestigung der beiden Maulteile genügt dann eine Gabel mit zwei Gabelschenkeln an dem Zugelement, wobei die Maulteile über einen entsprechenden Stift mit den Gabelschenkeln verbunden sind. Bei einem Herausnehmen des Zugelementes zusammen mit den Maulteilen können infolge der Ausbildung der Ausnehmung die Führungsschenkel in den Bereich zwischen die Gabelschenkel einfahren, so daß sie sich beim Herausnehmen des Zugelementes nicht störend auswirken. Somit kann dieses Zugelement zusammen mit den Maulteilen getrennt vom Schaft sterilisiert und gereinigt werden. Gleichzeitig ist auf einfache Weise auch eine Reinigung des Schaftinneren möglich.

Der Zusammenbau erfolgt wiederum auf einfache Weise, indem die beiden Maulteile etwa waagerecht angeordnet werden. Hierbei verschwinden die Führungsschenkel wieder zwischen den Gabelschenkeln und das Zugelement kann zusammen mit den Maulteilen in das Schaftrohr eingesetzt werden. Beim Eingleiten der Ringstücke in die Ausnehmung werden dann wiederum die Führungsschenkel aus ihrer versteckten Lage geschwenkt und gleiten in den Durchbruch ein, wobei die Maulteile um den Stift bzw. Gelenkbolzen drehen.

Das Ringstück kann im übrigen auch eine andere Ausgestaltung aufweisen. Beispielsweise kann hier ein Drehbolzen in eine sackschlitzartige Ausnehmung eingesetzt sein.

Damit beim Einsetzen der Maulteile in das Schaftrohr gleich eine Zentrierung erfolgt, so daß die Führungsschenkel die Durchbrüche finden, ist erfindungsgemäß eine Führungsklammer 31 vorgesehen, welche dem Schaftrohr aufgesetzt werden kann. Diese Führungsklammer besteht in den vorliegendem Ausführungsbeispiel aus zwei offenen Bögen, welche über Raststreifen miteinander verbunden sind. Zwischen den Bögen werden die Maulteile aufgenommen. Die Raststreifen besitzen wiederum Rastnasen, deren Lage axial mit den Führungsschenkeln übereinstimmt. Diese Rastnasen klipsen dann in die Durchbrüche ein, so daß von dem Schaftinneren her die Führungsschenkel in jedem Fall den Durchbruch finden. Dies ist eine bevorzugte Ausführungsform der Erfindung.

Eine andere Möglichkeit der Zentrierung bzw. der Erleichterung des Einsetzens der Maulteile in das Schaftrohr besteht darin, daß an dem Zugelement ein Kolbenstück vorhanden ist, in welches zumindest eine Führungsnut eingeformt ist. Diese Führungsnut spielt dann beim Einsetzen des Kolbenstückes in das Schaftrohr mit einer entsprechenden Führungsnase in dem Schaftrohr zusammen. Hierdurch wird gleichzeitig eine Verdrehsicherung gewährleistet.

Insgesamt ist das chirurgische Instrument außerordentlich einfach aufgebaut und leicht zu bedienen. Es läßt eine hohe Kraftübertragung auf die Maulteile zu, so daß die Schneidwirkung verbessert ist. Hervorzuheben ist vor allem das leichte Auseinandernehmen. Hierzu braucht lediglich das Zugelement von einem entsprechenden Zangen- bzw. Scherengriff od. dgl. gelöst zu werden.

Diesem Zweck dient vor allem auch eine weitere Ausgestaltung der Erfindung. Zur Bewegung der beiden Maulteile sind entsprechende Zangenschenkel vorgesehen, welche durch die menschliche Hand geöffnet und geschlossen werden können. An dem einen Zangenschenkel ist das Schaftrohr, meist in einer Halterung, festgelegt, während das Zugelement in der Regel mit dem anderen Zangenschenkel verbunden ist. Im vorliegenden Ausführungsbeispiel weist das Zugelement eine Mitnehmerkugel auf, welche in Gebrauchslage in einer Kugelaufnahme in einem Hals dieses Zangenschenkels sitzt. Beide Zangenschenkel drehen dann um einen gemeinsamen Drehpunkt.

Damit nun das Zugelement auf einfache Weise von seinem Zangenschenkel gelöst werden kann, andererseits aber nicht bei einer normalen Betätigung des chirurgischen Instrumentes aus seiner Halterung gleitet, sollte eine Riegeleinrichtung vorgesehen sein, welche die Bewegung der beiden Zangenschenkel zueinander begrenzt. Die Begrenzung der Bewegung bewirkt, daß bei normaler Betätigung der Zangen die Mitnehmerkugel in jeder Endlage in der Kugelaufnahme verbleibt, daß aber beim Lösen dieser Riegeleinrichtung der eine Zangenschenkel soweit geschwenkt werden kann, daß die Mitnehmerkugel einfach aus der Kugelaufnahme gleitet und dann das Zugelement zusammen mit den Maulteilen aus dem Schaftrohr gezogen werden kann.

In einer bevorzugten Ausführungsform weist diese Riegeleinrichtung im wesentlichen einen Rundbogen auf, der ebenfalls um den Drehpunkt der beiden Zangenschenkel dreht. Zu seiner Aufnahme ist in den einen Zangenschenkel eine Führungsrinne eingeformt, in welcher der Rundbogen gleitet. In der einen Endlage, in welcher die Bewegung der beiden Zangenschenkel zueinander begrenzt werden soll, ragt der Rundbogen mit einem Anschlagbolzen über eine Anschlagfläche hinaus, so daß der eine Zangenschenkel nur bis zu diesem Anschlagbolzen bewegt werden kann. Wird dagegen der Rundbogen in seiner Lage verändert, so verschwindet dieser Anschlagbolzen in dem Zangenschenkel. D.h., der Zangenschenkel kann bis zur Anschlagfläche geschwenkt werden, wobei in dieser Endlage die Mitnehmerkugel aus der Kugelaufnahme gleiten kann.

Bevorzugt soll der Rundbogen in seiner Endlage, in der er mit einem Anschlagbolzen über die Anschlagfläche hinaussteht, lösbar festgelegt sein. Dies geschieht auf einfache Weise, indem der Rundbogen in dieser Endlage eine im Zangenschenkel vorgesehene Rastnase hintergreift. Durch einen einfachen Druck auf eine Lasche od. dgl. an dem Rundbogen kann der Rundbogen aus dieser Rastnasenlagerung herausgeschoben werden, so daß er dann frei ist und um den Drehpunkt drehen kann.

Die vorliegende Erfindung befaßt sich auch mit einer weiteren Ausgestaltung der Zangenschenkel bzw. deren Festlegung zueinander, die bevorzugt bei dem vorliegenden chirurgischen Instrument Anwendung finden soll. Allerdings soll die vorliegende Erfindung nicht auf diese ausschließliche Anwendung beschränkt sein, sondern die nachfolgend beschriebene Ausgestaltung einer Sperreinrichtung ist auf viele chirurgische Instrumente übertragbar, die mit Zangen- bzw. Scherengriffen arbeiten.

Der erfinderische Gedanke bezieht sich vor allem auf die Möglichkeit, die Zangenschenkel in einem bestimmten Verhältnis zueinander festzulegen.

Hierfür sind Sperreinrichtungen bekannt, wobei beispielsweise zwei Sperrleisten an jedem Schenkel befestigt sind und sich über- bzw. untergreifen, wobei jeweiligen Zähne miteinander in Eingriff stehen. Nachteilig ist dabei, daß zum Lösen der Sperreinrichtung eine zweite Hand notwendig ist.

Eine andere Sperreinrichtung besteht aus einer Sperrleiste, welche einerseits gelenkig mit dem einen Zangenschenkel verbunden ist und andererseits von einer Feder gegen einen Rastzahn an dem anderen Zangenschenkel gedrückt wird. Wird davon ausgegangen, daß der Benutzer die Zange bzw. Schere mit Daumen und Zeigefinger bedient, so kann diese Sperreinrichtung nur mit der anderen Hand gelöst werden.

Andere Sperreinrichtungen sind entweder schwer zu bedienen oder aber sehr kompliziert aufgebaut.

Die vorliegende weitere Ausgestaltung der Erfindung entwickelt einen Zangen- bzw. Scherengriff, bei welchem die Sperreinrichtung sehr einfach aufgebaut und vor allem sehr leicht mit nur einer Hand zu betätigen ist und zwar mit der Hand, die auch den Zangen- bzw. Scherengriff selbst betätigt.

Diesem Zweck dient, daß an die Sperrleiste ein Hebelschenkel ggfs. mit einer Fingermulde anschließt.

Das Drehen der Sperrleiste, welche die Sperrzähne außer Eingriff mit dem Einrastzahn bringt, kann mittels eines Fingers derjenigen Bedienhand bewirkt werden, welche auch den Zangen- bzw. Scherengriff hält. Meist wird dies mittels dem Mittelfinger, Ringfinger oder kleinen Finger geschehen.

Bevorzugt soll die Sperrleiste unter dem Druck eines Kraftspeichers stehen, wobei der Druck bewirkt, daß die Sperrleiste in Eingriff mit dem Einrastzahn bleibt. Dies bedeutet, daß vom Bediener zum Lösen der Sperreinrichtung lediglich eine Bewegung eines Fingers in eine Richtung durchgeführt werden muß. Die Einrastbewegung wird dann von dem Kraftspeicher übernommen.

Die Sperrleiste kann dem Zangenschenkel mit dem Einrastzahn anliegen oder ihn umgreifen. Bevorzugt wird jedoch in den Zangenschenkel ein Schlitz eingeformt, den die Sperrleiste durchgreift. Hierdurch stört die Sperrleiste die normale Zangen- bzw. Scherengrifftätigkeit am wenigsten. Ferner wird hierdurch die Sperrleiste gesicherter geführt.

Greift die Sperrleiste den Zangenschenkel von außen her an, so ist dort dem Zangenschenkel ein zusätzlicher Einrastzahn angeformt. Durchgreift die Sperrleiste allerdings in dem bevorzugten Ausführungsbeispiel den Zangenschenkel, so genügt es, wenn dort eine Schlitzkante keilförmig zu einem Einrastzahn geformt ist.

Gedacht ist auch an eine klammerförmige Aufnahme für die Sperrleiste an dem anderen Zangenschenkel, in welche die Sperrleiste eingreift.

Für die Lagerung der Sperrleiste an dem anderen Zangenschenkel ist an einen einfachen Gelenkstift gedacht, um den herum die Sperrleiste drehen kann. Damit ein günstiger Angriff des als Federstreifen ausgebildeten Kraftspeichers erfolgt, soll die Anlenkung mittels einer hakenförmigen Anformung erfolgen, welche zwischen sich und der eigentlichen Sperrleiste eine Eingriffsmulde ausbildet, in die der Kraftspeicher eingreifen und gegen den Haken drücken kann. Dabei ist in einem bevorzugten Ausführungsbeispiel der Kraftspeicher als ein einfacher Federstreifen ausgebildet, der an einer Innenfläche des Zangenschenkels festliegt. Gegen diesen Federstreifen stützt sich dann eine Stirnfläche des Hakens oberhalb des Gelenkstiftes ab, so daß die Sperrleiste unter einer Vorspannung in eine Richtung zum Einrastzahn hin steht.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in

Fig. 1 einen Längsschnitt durch ein erfindungsmäßes chirurgisches Instrument;

Fig. 2 eine Explosionsdarstellung von Teilen des chirurgischen Instrumentes gem. Fig. 1, teilweise gebrochen dargestellt ;

Fig. 3 einen Längsschnitt durch einen Teilbereich des chirgischen Instrumentes gem. Fig. 1 in einer weiteren Gebrauchslage;

Fig. 4 einen Längsschnitt durch einen Teilbereich des chirurgischen Instrumentes gem. Fig 1 in zwei unterschiedlichen Gebrauchslagen;

Fig. 5 einen teilweise dargestellten Längsschnitt durch ein weiteres Ausführungsbeispiel des chirurgischen Instrumentes gem. Fig. 1;

Fig. 6 einen teilweise gebrochen dargestellten Ausschnitt aus Fig. 5 um 90 Grad gedreht;

Fig. 7 einen teilweise dargestellten Längsschnitt durch ein weiteres Ausführungsbeispiel eines chirurgischen Instrumentes gem. Fig. 1;

Fig. 8 einen Längsschnitt durch einen Teilbereich eines weiteren Ausführungsbeispiels eines chirurgischen Instrumentes;

Fig. 9 einen teilweise dargestellten Längsschnitt durch ein weiteres Ausführungsbeispiel eines chirurgischen Instruments ähnlich Figur 7;

Fig. 10 einen Längsschnitt durch einen Teilbereich eines weiteren Ausführungsbeispiels eines chirurgischen Instrumentes;

Fig. 11 eine Draufsicht auf einen Teil des chirurgischen Instrumentes gemäß Figur 10;

Fig. 12 eine Draufsicht auf ein Maulteil des chirurgischen Instrumentes geäß Figur 10;

Fig. 13 eine Draufsicht auf einen erfindungsgemässen Zangen- bzw. Scherengriff in Öffnungslage;

Fig. 14 Querschnitte durch verschiedene Ausführungsformen von Rastelementen;

Fig. 15 eine Draufsicht auf ein weiteres Ausführungsbeispiel eines erfindungsgemäßen Zangen- bzw. Scherengriffes in Öffnungslage;

Fig. 16 eine Draufsicht auf das Ausführungsbeispiel des erfindungsgemäßen Zangen- bzw. Scherengriffs gemäß Figur 15 in einer weiteren Gebrauchslage;

Fig. 17 eine vergrößert dargestellte Draufsicht auf einen Teilbereich des erfindungsgemäßen Zangen- bzw. Scherengriffes entsprechend Figur 13;

Fig. 18 eine vergrößert dargestellte Draufsicht auf den Teilbereich des Zangen- bzw. Scherengriffs entsprechend Figur 17 in einer weiteren Gebrauchslage;

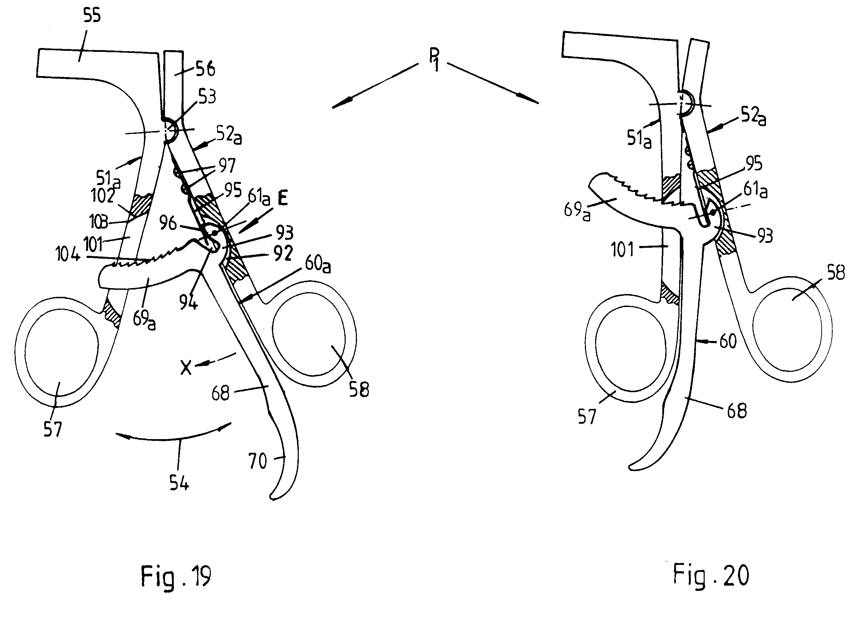

Fig. 19 eine Draufsicht auf ein weiteres Ausführungsbeispiel eines erfindungsgemäßen Zangen- bzw. Scherengriffs in Öffnungslage;

Fig. 20 eine Draufsicht auf den Zangen- bzw. Scherengriff gemäß Figur 19, jedoch in Schließlage;

Fig. 21 und 22 Draufsichten auf ein weiteres Ausführungsbeispiel eines erfindungsgemäßen Zangen- bzw. Scherengriffs entsprechend den Fig. 19 und 20.

Ein erfindungsgemäßes chirurgisches Instrument R, wie beispielsweise eine Zange oder Schere für die Endoskopie, weist ein Schaftrohr 1 auf, in welchem eine Zugstange 2 geführt ist. Im vorliegenden Ausführungsbeispiel ist die Zugstange 2 im wesentlichen aus einer Stange 3 sowie einem Kolbenstück 4 zusammengesetzt, wobei das Kolbenstück 4 einen größeren Durchmesser aufweist als die Stange 3. Einends ist an die Stange 3 über ein Anschlußstück 5 eine Mitnehmerkugel 6 angeformt, während andernends in dem Kolbenstück 4 zwei bewegbare Maulteile 7 und 8 lagern. An Stelle der Maulteile 7 und 8 können hier auch Scherenteile, Zangenschenkel oder ähnliche Instrumententeile festgelegt sein.

Die Mitnehmerkugel 6 dient zum Anschluß an einen Scheren- oder Zangengriff in bekannter Ausführung, wobei dann die Mitnehmerkugel 6 in einer entsprechenden Ausnehmung des Scherenteiles sitzt und das Anschlußstück 5 einen Schlitz durchgreift. Von diesem Scherengriff ist ferner ein weiteres Führungsstück 9 gezeigt, welches ebenfalls von der Stange 3 durchsetzt ist.

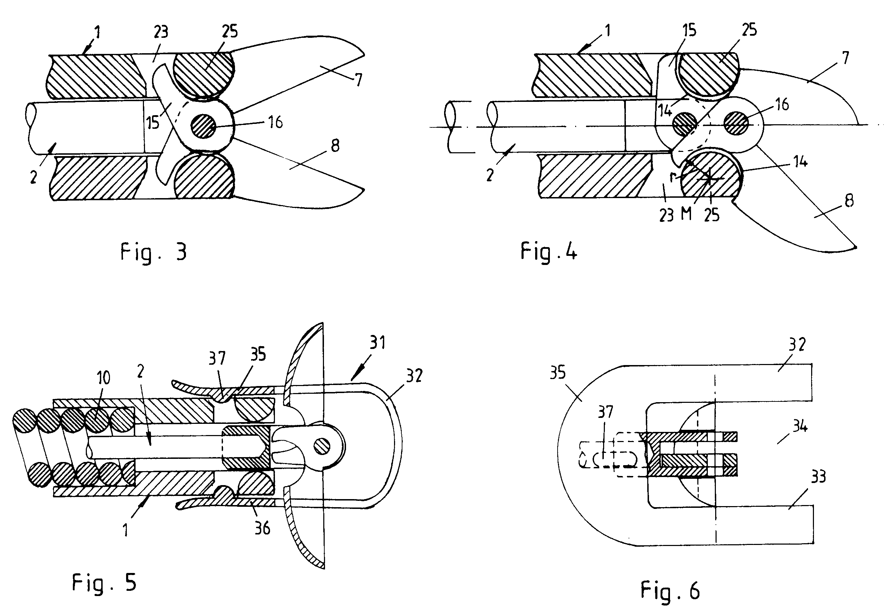

Sowohl für das Führungsstück 9 bzw. das gesamte Scherenteil als auch für die Zugstange 2 sind vielfältige Ausführungsformen denkbar und bekannt und sollen von der vorliegenden Erfindung umfaßt werden. Beispielsweise kann die Stange 3 auch durch ein flexibles Zugseil ersetzt werden. Ferner ist in Fig. 5 erkennbar, daß zur Rückführung der Zugstange 2 Federn 10 in dem Schachtrohr 1 angeordnet sein können. Hier sind, wie gesagt, vielfältige Ausführungsformen bekannt.

Wesentlich im Rahmen der vorliegenden Erfindung ist dagegen die Ausgestaltung der Maulteile 7 und 8, deren Anordnung und Verbindung an dem Kolbenstück 4 sowie der vordere Bereich des Schachtrohres 1 zum Führen dieser Maulteile 7 bzw. 8. Jedes Maulteil 7, 8 weist eine Schneid- oder Klemmkante 11 auf, welche generell als Arbeitskante bezeichnet werden kann. Von der Spitze 12 des Maulteils 7, 8 schließt dann eine gebogenen Rückwand 13 an, in welche eine halbkreisförmige Ausnehmung 14 eingeformt ist. Durch diese halbkreisförmige Ausnehmung 14 wird ein Führungsschenkel 15 ausgebildet, der später näher beschrieben ist.

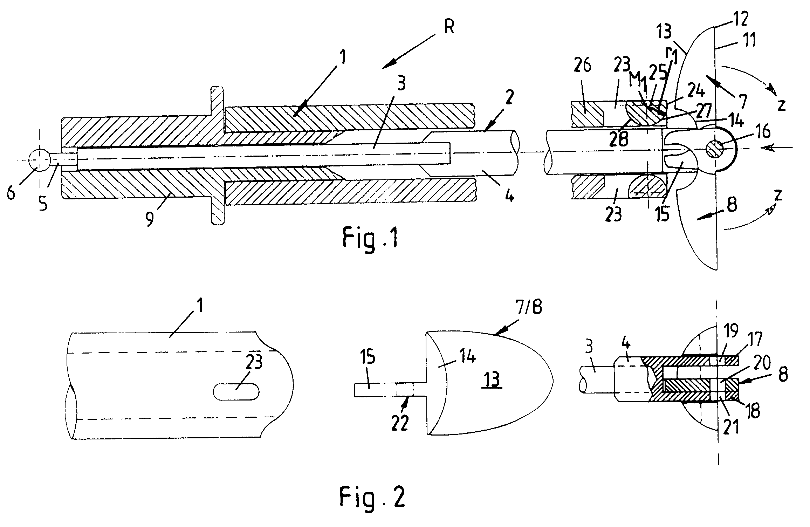

Beide Maulteile 7 und 8 sind im Bereich ihrer Führungsschenkel 15 bzw. neben den halbkreisförmigen Ausnehmungen 14 über einen Stift 16 mit dem Kolbenstück 4 verbunden, wobei hierzu in dem Kolbenstück 4, wie in Fig.2 gezeigt, eine Gabel aus zwei Gabelschenkeln 17 und 18 vorgesehen ist. Diese beiden Schenkel 17 und 18 nehmen zwischen sich die Maulteile 7 und 8 auf, so daß dann lediglich der Stift 16 durch die entsprechenden Gelenkbohrungen 19, 20 bzw. 21 der Schenkel 17, 18 bzw. der Maulteile 7 und 8 eingesetzt werden muß.

In Fig. 2 ist ferner eine Rückansicht eines Maulteiles 7/8 gezeigt, welches nur als Ausführungsbeispiel gedacht sein soll. Hierbei ist das Maulteil 7/8 im wesentlichen halbschalenförmig ausgebildet, so daß sich eine teilkalottenartige Rückwand 13 ausbildet. Daran schließt sich dann die Ausnehmung 14 an, welche jedoch zum Führungsschenkel 15 hin lediglich im Bereich eines Anschlußstreifens 22 vorgesehen ist.

In den Figuren 1 und 2 ist ferner erkennbar, daß sich im Schaft 1 beidseits Durchbrüche 23 befinden, welche der später erwähnten Aufnahme der Führungsschenkel 15 dienen. Zu einem Schaftrohrrandrand 24 hin sind die Durchbrüche 23 von einem, querschnittlich gesehen, halbkreisförmigen Ringstück 25 begrenzt, das in der entsprechenden Form aus der Wand 26 des Schaftrohres 1 herausgeformt ist. Dieses Ringstück 25 bildet zum Schaftrohrrand 24 hin, eine trichterförmige Einzugswand 27, während es den Durchbruch 23 ebenfalls trichterförmig erweitert, durch eine entsprechende Gleitkante 28 freigibt.

Die Funktionsweise der vorliegenden Erfindung ist folgende:

Zu Reinigungszwecken bzw. zum Desinfizieren wird die Mitnehmerkugel 6 oder eine ähnliche Halterung von dem entsprechenden Griffteil gelöst und die Zugstange 2 zu- sammen mit den Maulteilen 7 und 8 aus dem Anschlußstück 9 bzw. dem Schaftrohr 1 herausgezogen. Nunmehr kann sowohl das Schaftrohr in seinem Inneren wie auch die Zugstange 2 mit den Maulteilen 7 und 8 intensiv und in allen Bereichen gereinigt werden.

Zum Zusammenbau wird die Zugstange 2 wieder in das Schaftrohr 1 bzw. das Führungsstück 9 eingesetzt, wobei sich die Maulteile 7 und 8 in der in Fig. 1 gezeigten Gebrauchslage befinden. Dabei verschwinden die Führungsschenkel 15 beider Maulteile 7 und 8 zwischen den Gabelschenkeln 17 und 18, so daß sie zwischen den Ringstücken 25 durchgreifen können.

Sobald sich die Führungsschenkel 15 beider Maulteile 7 und 8 innerhalb der lichten Weite der Ringstücke 25 befin- den, schlägt die Rückwand 13 jedes Maulteiles 7 bzw. 8 an dem Schaftrohrrand 24 an, so daß die Maulteile 7 und 8 in Schließrichtung z bewegt werden. Hierbei öffnet sich die Ausnehmung 14 jedes Maulteiles 7 und 8 zu dem entsprechendem Ringstück 25 hin, wobei die Einzugswand 27 in die Ausnehmung 14 eingleitet, während der Führungsschenkel 15 der Gleitkante 28 aufgleitet und in den Durchbruch 23 einfährt. Diese unterschiedlichen Gebrauchslagen sind in den Figuren 3 und 4 stufenweise dargestellt. In Fig.4 unten ist erkennbar, wie das Ringstück 25 in die halbkreisförmige Ausnehmung 14 eingreift. Diese umschließt das Ringstück 25 zumindest teilweise.

In einer einer weiteren Gebrauchslage gem. Fig. 3 sind die Führungsschenkel 15 bereits weitgehend in die Durchbrüche 23 eingefahren und die Maulteile 7 und 8 auf dem Wege zu einer Schließstellung. Diese Schließstellung ist letztendlich in Fig. 4 oben für ein Maulteil 7 dargestellt. Dabei sitzt der Führungsschenkel 15 in dem Durchbruch 23, während das Ringstück 25 in der Ausnehmung 14 auf das Maulteil 7 drückt. Hierdurch ergibt sich eine sehr günstige Kraftverteilung.

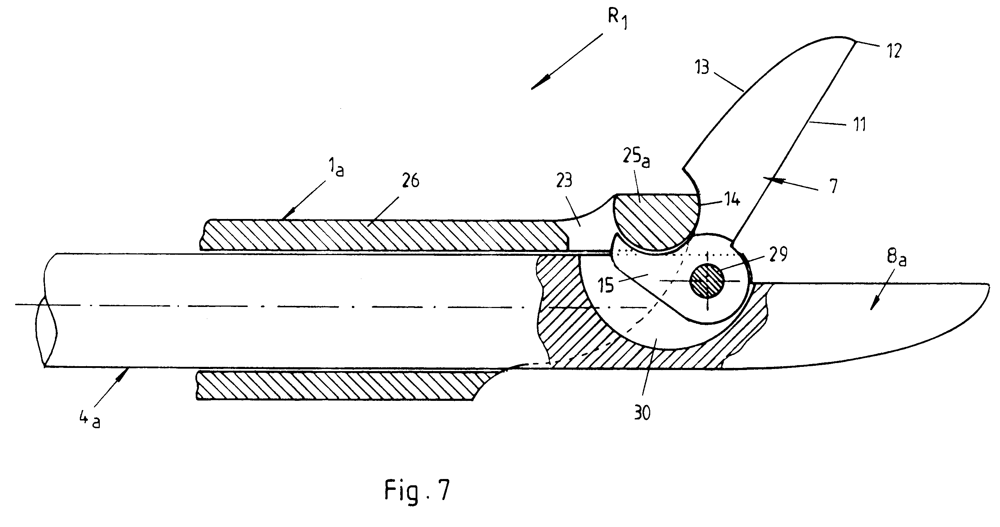

In einem weiteren Ausführungsbeispiel R1 der Erfindung gem. Fig. 7 ist dem entsprechenden Kolbenstück 4a ein Maulteil 8 a einstückig angeformt. Dieses feststehende Maulteil 8a ist über einen Gelenkbolzen 29 mit dem bewegbaren Maulteil 7 verbunden, welches, wie oben beschrieben, ausgestaltet ist. Damit das feststehende Maulteil 8a zumindest den Führungsschenkel 15 des Maulteiles 7 aufnehmen kann, ist in dem feststehenden Maulteil 8a eine Ausnehmung 30 ausgebildet.

In diesem Ausführungsbeispiel braucht in der Wand 26 des Schaftrohres 1a nur ein Durchbruch 23 für einen Führungsschenkel 15 vorgesehen sein. Deshalb ist hier auch zum Schaftrohrrand 24 hin nur ein Ringstück 25a ausgebildet.

Beim Herausziehen des Kolbenstückes 4a verschwindet der Führungsschenkel 15 in der Ausnehmung 30 und bildet so kein Hindernis für die Herausnahme. Beim Einsetzen dagegen schlägt das Ringstück 25a in der Ausnehmung 14 an, so daß die Ausnehmung 14 dieses Ringstück 25a umfaßt, wobei der Führungsschenkel 15 in den Durchbruch 23 einfährt und das Maulteil 7 um den Gelenkbolzen 29 gedreht wird.

In der Praxis hat sich gezeigt, daß insbesondere bei Verwendung von flexiblen Zugseilen anstelle von starren Zugstange 2 das Suchen der Durchbrüche 23 Schwierigkeiten macht. Um diesem Nachteil zu begegnen, ist dem Schachtrohr 1 gem. den Figuren 5 unnd 6 eine Führungsklammer 31 aufgesetzt. Diese Führungsklammer 31 bildet durch zwei offene Bögen 32 und 33 einen offenen U-förmigen Raum aus, in welchem sich die Maulteile 7 und 8 bewegen können. Beide Bögen 32 und 33 sind mittels Raststreifen 35 und 36 miteinander verbunden, wobei an jedem Raststreifen 35 und 36 nach innen eine Rastnase 37 angeformt ist, welche in den Durchbruch 23 einrasten kann. Hierdurch wird die Führungsklammer 31 lagefixiert, so daß auch die Maulteile 7 und 8 in den Raum 34 bezüglich des Schaftrohres 1 genau geführt sind. Da sich die Rastnasen 37 ebenso wie die Führungsschenkel 15 außermittig befinden, wird so gewährleistet, daß die Führungsschenkel 15 in die Durchbrüche 23 eingreifen und dabei die Rastnasen 37 aus den Durchbrüchen 23 verdrängen, so daß die Führungsklammer 31 abgenommen werden kann.

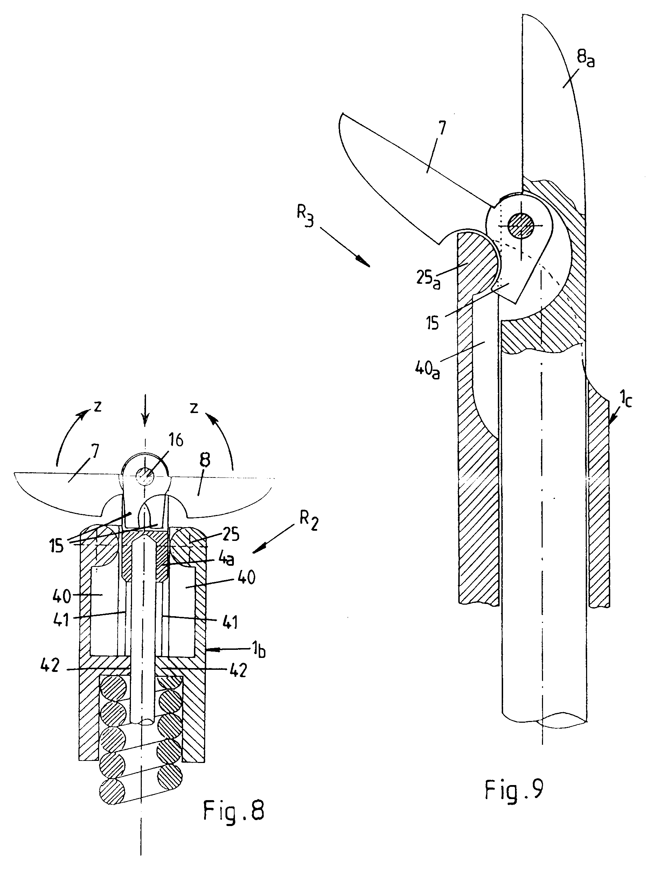

In dem weiteren Ausführungsbeispiel eines erfindungsgemäßen chirurgischen Instrumentes R2 gemäß Figur 8 ist erkennbar, daß ein entsprechendes Schaftrohr 1b keine Durchbrüche 23 für die entsprechenden Führungsschenkel 15 aufweist. Das Schaftrohr 1b ist somit nach außen hin eine geschlossene Hülse, so daß sich auch in einem Durchbruch kein Schmutz, Gewebereste od. Blut ansammeln kann.

Damit dennoch die Maulteile 7 und 8 mit den Ringstücken 25 in entsprechender Weise zusammenwirken, sind in das Schaftrohr 1b von innen her Nuten 40 eingeformt, in welche die Führungsschenkel 15 eingreifen können. Dabei ist selbstverständlich denkbar, daß die Führungsschenkel 15 wesentlich kürzer als dargestellt ausgebildet sind.

Ferner ist in Figur 8 anstelle der oben beschriebenen Führungsklammer 31 eine andere Zentriermöglichkeit bzw. Führung eines Kolbenstückes 4a vorgesehen, welche eine Führungsklammer 31 entfallen läßt. Das Kolbenstück 4a ist als eine verlängerte Hülse ausgebildet. Wobei ihr beidseits von außen her Führungsnuten 41 eingeformt sind. In diese Führungsnuten 41 greifen Führungsnase 42 ein, welche dem Schaftrohr 1b innen angeformt sind. Diese Führungsnasen 42 bewirken zusammen mit den Führungsnuten 41 eine Zentrierung des Kolbenstückes 4a und damit auch der Maulteile 7 und 8. Ferner bewirken sie eine Verdrehsicherheit.

In der weiteren Ausführungsform R3 eines erfindungsgemäßen chirurgischen Instrumentes gemäß Figur 9 ist lediglich gezeigt, daß auch hier ein Schaftrohr 1c keinen Durchbruch aufweist, wie dieser noch gemäß Figur 7 vorgesehen war. Statt dessen ist auch dort dem Schaftrohr 1c nach dem Ringstück 25a von innen her eine Nut 40a eingeformt, in welche der Führungsschenkel 15 einleiten kann.

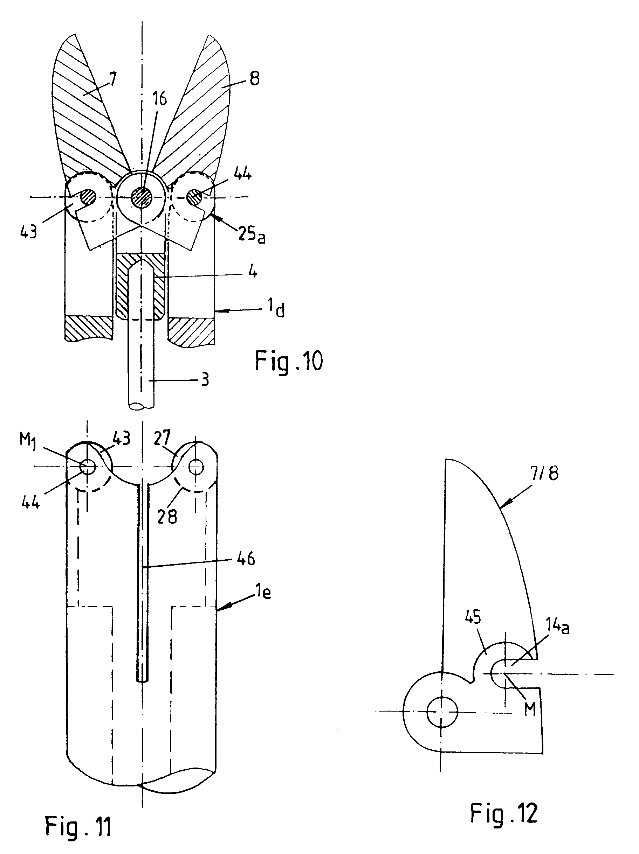

Die Figuren 10 bis 12 betreffen eine andere Ausgestaltung der Maulteile 7/8 und deren Führung an einem Ringstück 25a. Das Ringstück 25a weist hier zwar auch eine Einzugswand 27 und eine Gleitkante 28 auf, allerdings sind pro Ringstück 25a zwei scheibenförmige Elemente 43 vorgesehen, zwischen denen ein Drehbolzen 44 mit wesentlich geringererem Durchmesser angeordnet ist.

Dieser Drehbolzen 44 sitzt in Gebrauchslage in einer Ausnehmung 14a, welche als Sackschlitz ausgebildet ist. Der Grund dieser Ausnehmung 14a ist ebenfalls halbkreisförmig, so daß auch hier ein Mittelpunkt M ausgebildet ist, der in Gebrauchslage in etwa mit einem Mittelpunkt M1 des Drehbolzens 44 übereinstimmt.

Auch die scheibenförmigen Elemente 43 sitzen in Gebrauchslage in entsprechenden Aussparungen 45 der Maulteile 7/8 und drehen in diesen. Hierdurch findet eine gute Abstützung der Maulteile 7/8 statt.

In Figur 11 ist des weiteren erkennbar, daß in das Schaftrohr 1d eine Dehnungsfuge 46 eingeschnitten ist, welche eventuelle Passungenauigkeiten ausgleicht.

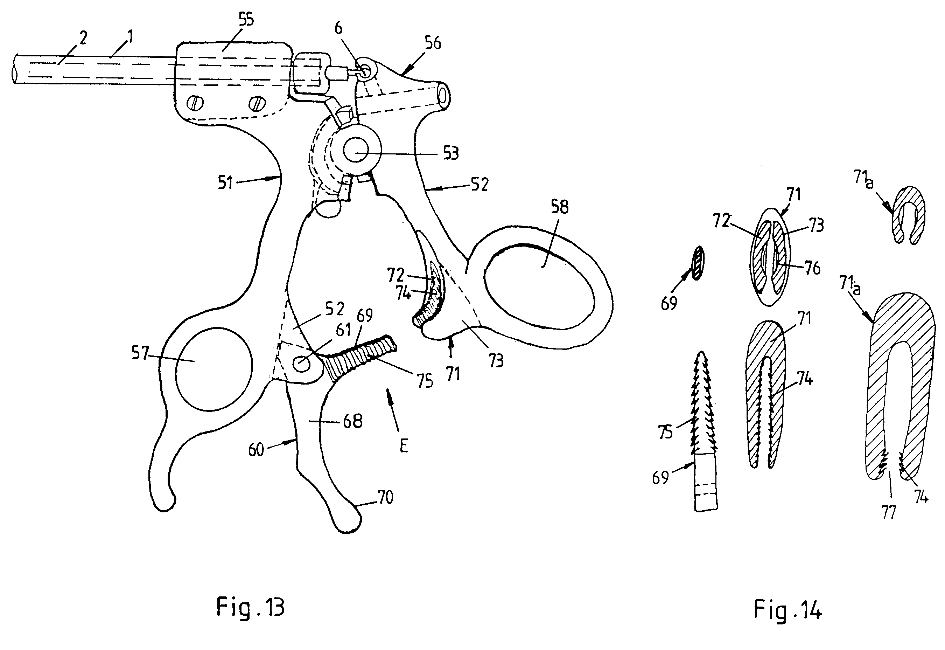

Ein erfindungsgemäßer Zangen- bzw. Scherengriff P für ein chirurgisches Instrument R weist gemäß Figur 13 zwei Zangenschenkel 51 und 52 auf, welche an einem Drehpunkt 53 miteinander gelenkig verbunden sind. Um den Drehpunkt 53 herum erfolgt eine Öffnungs- bzw. Schließbewegung in Richtung des Doppelpfeiles 54. Derartige Zangen- bzw. Scherengriffe finden auch ihren Einsatz bei chirurgischen Instrumenten, wie diese in der Europäischen Patentschrift 0 279 358 gezeigt sind. In der Regel schließt an eine entsprechende Halterung 55 des Zangenschenkels 51 eine eigentliche Arbeitseinrichtung des chirurgischen Instrumentes, insbesondere das Schaftrohr 1, an, welches dann von der Zugstange 2 durchzogen ist, die über die Mitnehmerkugel 6 mit einem Hals 56 des anderen Zangenschenkels 2 verbunden ist.

Zur Betätigung der Zangenschenkel 51 und 52, d.h., zur Durchführung der Öffnungs- bzw. Schließbewegungen in Richtung des Doppelpfeiles 54 sind im vorliegenden Ausführungsbeispiel Griffösen 57 und 58 jedem Zangeschenkel 51 bzw. 52 angeformt. Anstelle der Griffösen 57 und 58 können auch an dem Zangenschenkeln 51 und 52 beispielsweise einfache Griffrillen od. dgl. vorgesehen sein.

Wesentlich ist, daß die Zangenschenkel 51 und 52 durch eine Sperreinrichtung E im Abstand zueinander festgelegt werden können. Diese Sperreinrichtung E weist einen Hebel 60 auf, der mit dem Zangenschenkel 51 über einen Gelenkstift 61 drehbar verbunden ist. Hierzu sind dem Zangenschenkel 51 zwei Laschen 52 angeformt, welche den Hebel 60 klammerartig umgreifen.

Die Sperrleiste 69 ist einer Aufnahme 71 zugeordnet, die sich an dem anderen Zangenschenkel 52 befindet. In diesem Ausführungsbeispiel gemäß Figur 13 besteht die Aufnahme aus zwei Klammerstreifen 72 und 73, welche dem Zangenschenkel 52 bevorzugt einstückig angeformt sind. Inwändig sind den beiden Klammerstreifen 72 und 73 jeweils Rastzähne 74 eingeformt, welche mit entsprechenden Rastzähnen 75 auf der Außenfläche der Sperrleiste 69 zusammenwirken.

Die Funktionsweise des erfindungsgemäßen Zangen- bzw. Scherengriffs ist folgende:

Zur freien Bewegung der beiden Zangenschenkel 51 und 22 wird die Sperreinrichtung E gelöst, indem über den Hebel 60 die Sperrleiste 69 aus dem Eingriffsbereich der Aufnahme 71 geschwenkt wird. Die Rastzähne 74 sind außer Eingriff mit den Rastzähnen 75. Durch den Bediener wird der Hebel 60 in der entsprechenden Stellung gehalten, wobei beispielsweise der kleine Finger des Bedieners in der Fingermulde 70 liegen kann.

Sollen die Zangenschenkel 51 und 52 in einem bestimmten Verhältnis zueinander festgelegt werden, so bringt der Bediener die Sperrleiste 69 in eine Position, in der diese in die Aufnahme 71 einfahren kann. Dabei rasten die Rastzähne 75 in den Rastzähnen 74 ein. Bei Bedarf kann diese Sperre wieder durch einen entsprechenden Druck auf den Hebelschenkel 78 aufgehoben werden.

In Figur 14 ist eine Draufsicht auf und ein Querschnitt durch die Sperrleiste 69 dargestellt. Neben ihr ist im Querschnitt die Aufnahme 71 zu erkennen und vor allem auch die Rastzähne 74. Darüber ist ein Querschnitt durch die Aufnahme 71 dargestellt, wobei vor allem eine ovale Innenform erkennbar ist, welche eine bessere Führung der im Querschnitt ovalen Sperrleiste 69 gewährleistet.

In einem weiteren Ausführungsbeispiel kann eine Aufnahme 71a auch nur nach einer Aufnahmeöffnung 77 mit wenigen Rastzähnen 74 belegt sein. Der entsprechende Querschnitt ist ebenfalls in Figur 14 darüber dargestellt.

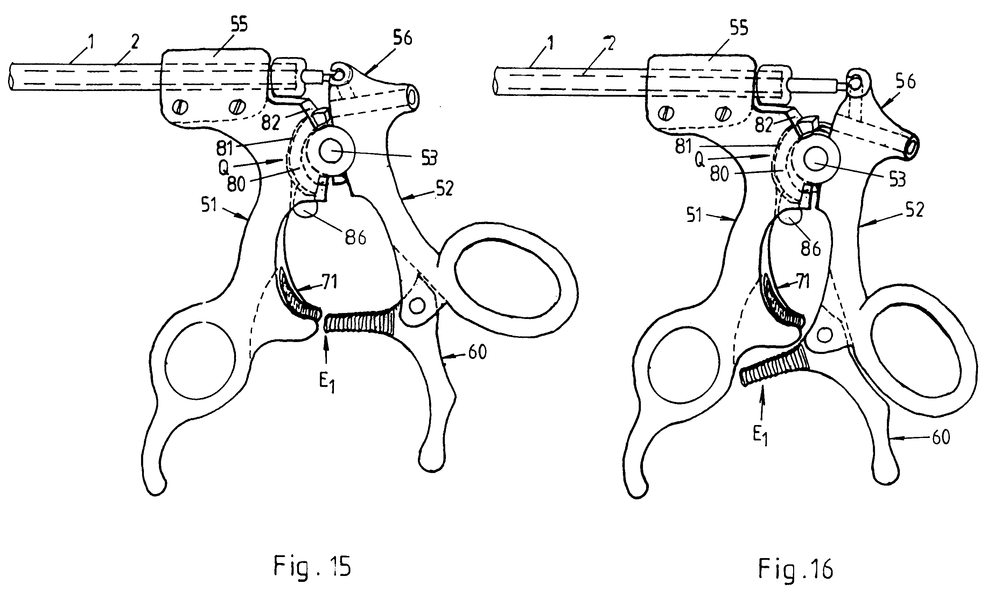

Die Figuren 15 und 16 verdeutlichen, daß die Sperreinrichtung E1 auch umgedreht angeordnet sein kann, d.h., daß sich der Hebel 60 an dem Zangenschenkel 52 und die Aufnahme 71 an dem Zangenschenkel 51 befindet. Auch diese Ausführungsform soll vom Erfindungsgedanken umfaßt sein, da sie mit der in Figur 13 gezeigten Ausführungsform äquivalent ist.

In den Figuren 15 bis 18 ist jedoch noch eine besonders bevorzugte Riegeleinrichtung Q dargestellt, welche zum eine gewährleistet, daß bei der Bewegung der Zangenschenkel 51 und 52 die Verbindung der Zugstange 2 an dem Hals 56 erhalten bleibt, daß andererseits aber diese Verbindung auf einfache Weise gelöst werden kann, damit eine Entnahme der Zugstange aus dem Schaftrohr 1 und damit eine Reinigung der entsprechenden Teile sehr leicht möglich ist.

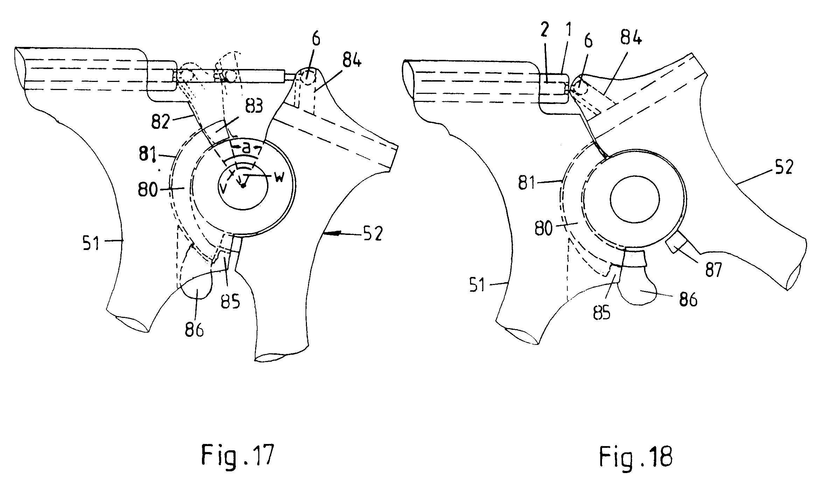

Hierzu weist die Riegeleinrichtung Q bevorzugt einen Rundbogen 80 auf, der um den Drehpunkt 53 angeordnet ist. Dieser Rundbogen 80 ist auch um diesen Drehpunkt 53 in einer entsprechenden Führungsrinne 81 bewegbar, wobei er allerdings, wie in Figur 17 gezeigt in einer bestimmten Gebrauchslage festgelegt werden kann. In dieser Gebrauchslage ragt aus einer Anschlagfläche 82 des Zangenschenkels 41 ein Anschlagbolzen 83 als Teil des Rundbogens 80 hervor und begrenzt somit eine Drehbewegung des Zangenschenkels 52 auf einen Winkel w. Dieser Winkel w ist so ausgelegt, daß sich die Mitnehmerkugel 6 nach wie vor so in einer Kugelaufnahme 84 befindet, daß sie nicht aus dieser Kugelaufnahme 84 herausgleiten kann. Dies ist gestrichelt in Figur 17 angedeutet. In dieser Lage schlägt übrigens der Rundbogen 80 andernends des Anschlagbolzens 83 an einer Rastnase 85 an und ist so drehsicher festgelegt.

Zum Lösen des Rundbogens 80 wird dieser über eine Lasche 86 angehoben, so daß er die Rastnase 85 überfahren kann, wie dies in Figur 18 dargestellt ist. Hierbei verschwindet der Anschlagbolzen 83 innerhalb der Führungsrinne 81 und bietet so keine Begrenzung mehr für die Drehbewegung des Zangenschenkels 52. Aus diesem Grunde kann der Zangenschenkel 53 eine Bewegung um den Winkel v vollziehen, wobei die Kugelaufnahme 84 in eine Stellung gerät, bei der die Mitnehmerkugel 6 aus der Kugelaufnahme 84 gleiten kann. Nunmehr kann die Zugstange 2 ohne weiteres aus dem Schaftrohr 1 entnommen werden.

Nach einer Reinigung aller Teile genügt es, wenn die Zugstange 2 in das Schaftrohr 1 eingeschoben wird, wobei dann die Mitnehmerkugel 6 ohne weiteres in die Kugelaufnahme 84 eingleitet. Nunmehr wird der Zangenschenkel 52 gegen den Zangenschenkel 51 bewegt, so daß es zu einem Schließen der beiden Zangenschenkel kommt. Dabei trifft ein Rückschubbolzen 87 auf den Rundbogen 80 und schiebt diesen zusammen mit der Lasche 86 in eine Rastlage, in welcher der Rundbogen 80 die Rastnase 85 hintergreift und der Anschlagbolzen 83 aus der Anschlagfläche 82 hervorsteht. Bei einer jetzt folgenden weiteren Bedienung der Zangenschenkel 51 und 52 kann die Kugelaufnahme 84 nicht in eine Lage geraten, bei die Mitnehmerkugel versehentlich aus der Kugelaufnahme 84 gleitet.

Der Rundbogen 80 kann auch wahlweise mit derselben Funktion im Zangenschenkel 52 angeordnet sein. In einem weiteren Ausführungsbeispiel einer Sperreinrichtung E ist in einem Zangenschenkel 52a eine Ausnehmung 92 eingeformt, in welcher ein einem Hebel 60a angeformter Haken 93 sitzt. Dieser Haken 93 durchsetzt endwärts ein Gelenkstift 61a, wobei der Haken 93 ferner mit dem Hebel 60a eine Eingriffsmulde 94 ausbildet, in welche in Gebrauchslage ein Federstreifen 95 eingreift. Gegen diesen Federstreifen 95 stützt sich eine Stirnfläche 96 des Hakens 93 ab, wobei durch den unter Vorspannung stehenden Federstreifen 95 eine Bewegung des Hebels 60a in Richtung x bewirkt wird. Der Federstreifen 95, welche über entsprechende Schrauben 97 an dem Zangenschenkel 52a festgelegt ist, drückt auf die Stirnfläche 96, so daß der Hebel 60a um den Gelenkstift 61a dreht.

Sollen die Zangenschenkel 51a und 52a in einem bestimmten Verhältnis zueinander festgelegt werden, so genügt es, wenn der Bediener seinen Gegendruck gegen den Federstreifen 95 aufhebt, so daß dieser Federstreifen 95 den Hebel 60a um den Gelenkstift 61a drehen kann und der Einrastzahn 103 in einen der Sperrzähne 104 einrastet. Bei Bedarf kann diese Sperre wieder durch einen entsprechenden Druck auf den Hebelschenkel 68 aufgehoben werden.

Der Haken 93 ist im übrigen in einem Bereich zwischen dem Hebelschenkel 68 und einer Sperrleiste 69 angeordnet. An dem Hebelschenkel 68 schließt noch die Fingermulde 70 an.

Die Sperrleiste 69a durchgreift einen Schlitz 101 in dem anderen Zangenschenkel 51a, wobei eine obere Schlitzkante 102 keilförmig ausgebildet ist und einen Einrastzahn 103 ausbildet. Mit diesem Einrastzahn 103 wirken Sperrzähne 104 auf der Oberseite der Sperrleiste 69a zusammen, wie dies insbesondere in Figur 2 gezeigt ist.

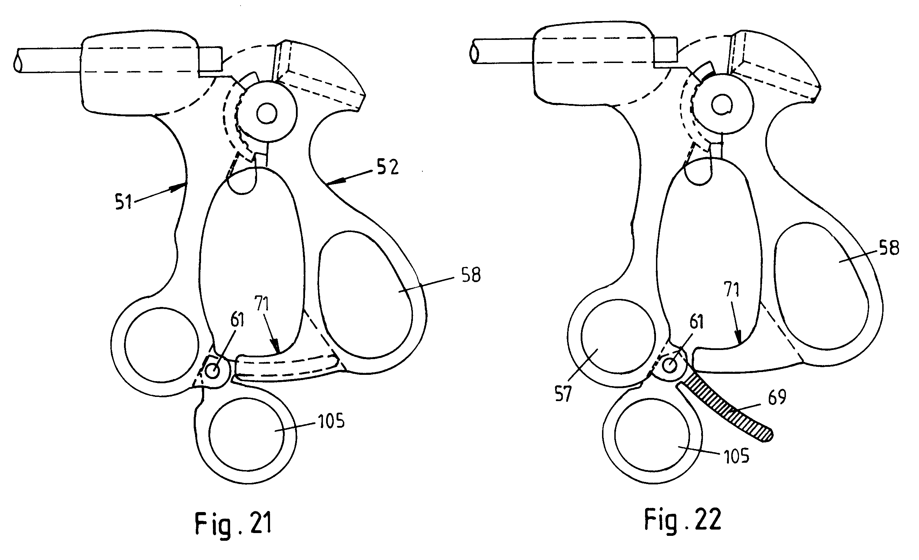

In den Figuren 21 und 22 ist zusätzlich gezeigt, daß statt einer Fingermulde am Hebelschenkel 68 eine Grifföse 105 vorgesehen ist, wodurch eine Bewegen des Hebelschenkels 68 sowohl nach vorne als auch nach hinten ohne Umsetzen des Fingers möglich ist.

## P O S I T I O N S Z A H L E N L I S T E

| | | | | | |
|---|---|---|---|---|---|
| 1 | Schaftrohr | 34 | Raum | | |
| 2 | Zugstange | 35 | Raststreifen | | |
| 3 | Stange | 36 | " | | |
| 4 | Kolbenstück | 37 | Rastnase | | |
| 5 | Anschlußstück | 38 | | | |
| 6 | Mitnehmerkugel | 39 | | | |
| 7 | Maulteil | 40 | Nut | | |
| 8 | Maulteil | 41 | Führungsnut | | |
| 9 | Führungsstück | 42 | Führungsnase | | |
| 10 | Feder | 43 | scheibenförmiges Element | | |
| 11 | Arbeitskante | 44 | Drehbolzen | M | Mittelpunkt v. 14 |
| 12 | Spitze | 45 | Aussparung | $M_1$ | Mittelpunkt v. 15 |
| 13 | Rückwand | 46 | Drehungsfuge | | |
| 14 | Ausnehmung | 47 | | | |
| 15 | Führungsschenkel | 48 | | R | chirurg. Instrument |
| 16 | Stift | 49 | | | |
| 17 | Schenkel | 50 | | r | Radius von 14 |
| 18 | " | 51 | | $r_1$ | Radius von 15 |
| 19 | Gelenkbohrung | 52 | | | |
| 20 | " | 53 | | | |
| 21 | " | 54 | | | |
| 22 | Anschlußstreifen | 55 | | | |
| 23 | Durchbruc | 56 | | | |
| 24 | Schaftrohrrad | 57 | | | |
| 25 | Ringstück | 58 | | | |
| 26 | Wand | 59 | | | |
| 27 | Einzugswand | 60 | | Z | Schließrichtung |
| 28 | Gleitkante | 61 | | | |
| 29 | Gelenkbolzen | 62 | | | |
| 30 | Ausnehmung | 63 | | | |
| 31 | Führungskammer | 64 | | | |
| 32 | Bogen | 65 | | | |
| 33 | " | 66 | | | |

| P O S I T I O N S Z A H L E N L I S T E | | | | | |
|---|---|---|---|---|---|
| 51 | Zangenschenkel | 84 | Kugelaufnahme | | |
| 52 | " | 85 | Rastnase | | |
| 53 | Drehpunkt | 86 | Lasche | | |
| 54 | Öffnungs- bzw. Schließbew. | 87 | Rückschubbolzen | | |
| 55 | Halterung | | | | |
| 56 | Hals | | | | |
| 57 | Grifföse | | | | |
| 58 | " | 91 | | $E_1$ | Sperreinrichtung |
| | | 92 | Ausnehmung | E | Sperreinrichtung |
| 60 | Hebel | 93 | Haken | | |
| 61 | Gelenkstift | 94 | Eingriffsmulde | | |
| 62 | Laschen | 95 | Federstreifen | | |
| | | 96 | Stirnfläche | P | Zangen- bzw. Scherengriff |
| | | 97 | Schraube | | |
| | | | | Q | Riegeleinrichtung |
| 68 | Hebelschenkel | 101 | Schlitz | x | Bewegung d. Hebels 60a |
| 69 | Sperrleiste | 102 | Schlitzkante | | |
| 70 | Fingermulde | 103 | Einrastzahn | | |
| 71 | Aufnahme | 104 | Sperrzähne | | |
| 72 | Klammerstreifen | 105 | Grifföse | | |
| 73 | " | | | v | Winkel |
| 74 | Rastzähne | | | w | Winkel |
| 75 | " | | | | |
| 76 | ovale Innenform | | | | |
| 77 | Aufnahmeöffnung | | | | |
| 80 | Rundbogen | | | | |
| 81 | Führungsrinne | | | | |
| 82 | Anschlagfläche | | | | |
| 83 | Anschlagbolzen | | | | |

**Patentansprüche**

1. Chirurgisches Instrument insbesondere für die Endoskopie mit einem schließbaren Maul aus Maulteilen wie Zangen- oder Scherenschenkeln, welche an einem Zugelement festgelegt sind, wobei zumindest ein Maulteil eine Drehachse mit dem Zugelement bildet und um diese Drehachse drehbar angeordnet ist und wobei das Zugelement ein Schaftrohr durchzieht,

dadurch gekennzeichnet,

daß zumindest einem Maulteil (7, 8) von außen her eine Ausnehmung (14, 14a) eingeformt ist, deren Mittelpunkt (M) um die Drehachse (16, 29) am Zugelement (2) dreht, wobei die Ausnehmung (14, 14a) in Gebrauchslage ein in der Wand des Schaftrohres (1) vorgesehenes Ringstück (25) zumindest teilweise umfängt und das Maulteil (7, 8) bei seiner Schließ- oder Öffnungsbewegung um dieses Ringstück (25) dreht.

2. Chirurgisches Instrument nach Anspruch 1, dadurch gekennzeichnet, daß die Ausnehmung (14) etwa halbkreisförmig ausgebildet ist und einen Führungsschenkel (15) ausbildet, der in einen Durchbruch (23) oder eine Nut (40) eingreift.

3. Chirurgisches Instrument nach Anspruch 2, dadurch gekennzeichnet, daß am Zugelement (2) eine Gabel mit zwei Gabelschenkeln (17, 18) ausgebildet ist, welche zwischen sich die Führungsschenkel (15) aufnehmen.

4. Chirurgisches Instrument nach Anspruch 3, dadurch gekennzeichnet, daß sowohl Gabelschenkel (17, 18) wie auch Führungsschenkel (15) von einem Stift (16) bzw. Gelenkbolzen (29) als Drehachse durchsetzt sind.

5. Chirurgisches Instrument nach Anspruch 2, dadurch gekennzeichnet, daß bei einem feststehenden Maulteil (8a) dieses eine Ausnehmung (30) zur Aufnahme des Führungsschenkels (15) des bewegbaren Maulteils (7) aufweist, welches in der Ausnehmung (30) über den Gelenkbolzen (29) mit dem feststehenden Maulteil (8a) verbunden ist.

6. Chirurgisches Instrument nach wenigstens einem der Ansprüche I - 5, dadurch gekennzeichnet, daß das Ringstück (25) etwa halbkreisförmig ausgebildet ist und vom Schaftrohrrand (24) her durch eine Einzugswand (27) eine trichterförmige Verengung und zum Durchbruch (23) bzw. der Nut (40) hin durch eine Gleitkante (28) eine trichterförmige Erweiterung bildet.

7. Chirurgisches Instrument nach Anspruch 6, dadurch gekennzeichnet, daß der Mittelpunkt (M 1) des Ringstücks (25) und der Mittelpunkt (M) der Ausnehmung (14) in Gebrauchslage übereinstimmen bzw. nahe beieinander liegen, wobei der Radius (r l) des Ringstückes (25) geringfügig geringer ist als der Radius (r) der Ausnehmung (14).

8. Chirurgisches Instrument nach wenigstens einem der Ansprüche 1 bis 5, dadurch gekennzeichnet, daß das Ringstück (25a) aus zwei scheibenförmigen Elementen (43) besteht, zwischen denen ein Drehbolzen (44) angeordnet ist, welcher in Gebrauchslage in einer sackschlitzartigen Ausnehmung (14a) sitzt, während die scheibenförmigen Elemente (43) von Aussparungen (45) in dem Maulteil (7, 8) aufgenommen sind.

9. Chirurgisches Instrument nach wenigstens einem der Ansprüche 1 - 8, dadurch gekennzeichnet, daß dem Schaftrohr (1) eine Führungsklammer (31) aufsetzbar ist, welche zwei Bögen (32, 33) ausbildet, zwischen denen die Maulteile (7, 8) aufgenommen sind, wobei die Führungskammer (31) dem Zentrieren der Führungsschenkel (15) gegenüber den Durchbrüchen (23) bzw. Nuten (40) dient.

10. Chirurgisches Instrument nach Anspruch 8, dadurch gekennzeichnet, daß die beiden Bögen (32, 33) über Raststreifen (35, 36) miteinander verbunden sind, welche Rastnasen (37) aufweisen, die in Gebrauchslage in die Durchbrüche (23) einrasten.

11. Chirurgisches Instrument nach wenigstens einem der Ansprüche 1 bis 8, dadurch gekennzeichnet, daß sich an dem Zugelement (2) ein Kolbenstück (4) befindet, in welchem zumindest eine Führungsnut (41) eingeformt ist, die mit einer Führungsnase (42) eingeformt ist, die mit einer Führungsnase (42) in dem Schaftrohr (1b) zusammenwirkt.

12. Chirurgisches Instrument nach wenigstens einem der Ansprüche 1 bis 11, dadurch gekennzeichnet, daß das Zugelement (2) andererseits des schließbaren Maules aus dem Schaftrohr (1) herausragt und an einem Hals (56) eines Zangenschenkels (52) od. dgl. lösbar festgelegt ist.

**13.** Chirurgisches Instrument nach Anspruch 12, dadurch gekennzeichnet, daß am Zugelement (2) eine Mitnehmerkugel (6) festliegt, welche in Gebrauchslage in einer Kugelaufnahme (84) in dem Hals (56) sitzt.

**14.** Chirurgisches Instrument nach Anspruch 12 oder 13, dadurch gekennzeichnet, daß das Schaftohr (2) an einer Halterung (55) befestigt ist, welche Teil eines weiteren Zangenschenkels (51) od. dgl. ist, der über einen Drehpunkt (53) mit dem Zangenschenkel (52) verbunden ist.

**15.** Chirurgisches Instrument nach Anspruch 14, dadurch gekennzeichnet, daß an dem weiteren Zangenschenkel (51) eine Riegeleinrichtung (Q) vorgesehen ist, welche die Bewegung des einen Zangenschenkels (52) im Verhältnis zum anderen Zangenschenkel (51) begrenzt.

**16.** Chirurgisches Instrument nach Anspruch 15, dadurch gekennzeichnet, daß im Zangenschenkel (51) eine Führungsrinne (81) für einen Rundbogen (80) vorgesehen ist, welcher um den Drehpunkt (53) dreht und in einer Endlage mit einem Anschlagbolzen (83) über eine Anschlagfläche (82) hinaussteht.

**17.** Chirurgisches Instrument nach Anspruch 16, dadurch gekennzeichnet, daß der Rundbogen (80) in dieser Endlage eine Rastnase (85) lösbar hintergreift.

**18.** Zangen- bzw. Scherengriff, insbesondere für ein chirurgisches Instrument nach wenigstens einem der Ansprüche 1 bis 17, mit zwei Zangenschenkeln od. dgl., welche über einen Drehpunkt miteinander gelenkig verbunden sind, und einer Sperreinrichtung, welche die beiden Zangenschenkel im Verhältnis zueinander festlegt, wobei die Sperreinrichtung eine Sperrleiste aufweist, welche gelenkig mit dem einen Zangenschenkel verbunden ist und über Sperrzähne mit einem oder mehreren Einrastzähnen an dem anderen Zangenschenkel in Wirkverbindung steht, dadurch gekennzeichnet, daß an die Sperrleiste (69, 69a) ein Hebelschenkel (68) ggfs. mit einer Fingermulde (70) oder Grifföse (105) anschließt.

**19.** Zangen- bzw. Scherengriff nach Anspruch 18, dadurch gekennzeichnet, daß der Hebelschenkel (68) etwa rechtwinklig von der Sperrleiste (69, 69a) nach unten abragt.

**20.** Zangen- bzw. Scherengriff nach Anspruch 18 oder 19, dadurch gekennzeichnet, daß die Sperrleiste (69a) unter dem Druck eines Kraftspeichers (95) in Eingriff mit zumindest einem Einrastzahn (74, 103) gehalten ist.

**21.** Zangen- bzw. Scherengriff nach einem der Ansprüche 18 bis 20, dadurch gekennzeichnet, daß an die Sperrleiste (69a) ein Haken (93) anschließt, welcher über einen Gelenkstift (61a) mit dem Zangenschenkel (52a) verbunden ist.

**22.** Zangen- bzw. Scherengriff nach Anspruch 21, dadurch gekennzeichnet, daß der Haken (93) in einer Ausnehmung (92) in dem Zangenschenkel (52a) sitzt.

**23.** Zangen- bzw. Scherengriff nach Anspruch 21 oder 22, dadurch gekennzeichnet, daß der Haken (93) mit der Sperrleiste (69a) eine Eingriffsmulde (94) ausbildet, in die ein als Federstreifen (95) ausgebildeter Kraftspeicher eingreift.

**24.** Zangen- bzw. Scherengriff nach Anspruch 23, dadurch gekennzeichnet, daß der Haken (93) zum Federstreifen (95) hin eine Stirnfläche (96) ausbildet, an welcher der Federstreifen (95) anliegt.

**25.** Zangen- bzw. Scherengriff nach Anspruch 18 oder 19, dadurch gekennzeichnet, daß der Sperrleiste (69) am anderen Zangenschenkel (52) eine Aufnahme (71) zugeordnet ist.

**26.** Zangen- bzw. Scherengriff nach Anspruch 25, dadurch gekennzeichnet, daß die Aufnahme (71) aus zwei Klammerstreifen (72, 73) besteht, deren Innenfläche mit Rastzähnen (74) belegt sind, die mit Rastzähnen (73) an der Sperrleiste (69) zusammenwirken.

**27.** Zangen- bzw. Scherengriff nach Anspruch 26, dadurch gekennzeichnet, daß die Klammerstreifen (72, 73) eine querschnittlich ovale Innenform (76) ausbilden, in welche die querschnittlich ovale Sperrleiste (69) eingreift.

Fig.1

Fig.2

EP 0 513 471 A2

Fig. 4

Fig. 6

Fig. 3

Fig. 5

Fig. 7

Fig.8

Fig.9

Fig. 10

Fig. 11

Fig. 12

Fig.14

Fig.13

Fig.16

Fig.15

Fig.18

Fig.17

20

Fig. 20

Fig. 19

Fig. 22

Fig. 21